# EUROPÄISCHE PATENTANMELDUNG

(11) **EP 2 494 968 A1**
(43) Veröffentlichungstag der Anmeldung: **05.09.2012**
(21) Anmeldenummer: 12001410.5
(22) Anmeldetag: 01.03.2012
(51) Int. Cl.: A61K 31/17, A61K 33/38, A61K 45/06, A61K 36/28, A61K 36/30, A61K 36/77, A61P 17/00, A61K 9/00, A61K 36/63

(54) **Zusammensetzung, insbesondere dermatologische Salbe mit Olea Europaea Fruchtöl und Silber**

(30) Priorität: 01.03.2011 DE 202011003362 U
(71) Anmelder: Dr. Theiss Naturwaren GmbH, 66424 Homburg (DE)
(72) Erfinder: Theiss, Peter Prof. Dr., 66484 Winterbach (DE)
(74) Vertreter: Zeitler - Volpert - Kandlbinder

(57) **Zusammenfassung**

Die vorliegende Erfindung betrifft eine Zusammensetzung, insbesondere dermatologische Salbe, enthaltend folgende Inhaltsstoffe (INCI): Olea Europaea Fruchtöl, Urea, Silber sowie Octyldodecanol, Echium Plantagineum Samenöl, Cardiospermum Halicaca.

## Beschreibung

Die Erfindung bezieht sich auf eine Zusammensetzung, insbesondere dermatologische Salbe.

Aus der EP 2 011 504 A1 ist ein Dermatikum zur Behandlung und/oder Pflege der Haut bei Neurodermitis bekannt. Dieses Dermatikum enthält Silberpartikel in Kombination mit Nachtkerzenöl zur Pflege trockener Hautzustände.

Der Erfindung liegt die Aufgabe zugrunde, eine Zusammensetzung, insbesondere eine dermatologische Salbe anzugeben, welche hautpflegend und reizmindernd sowie schnell wirksam ist.

Diese Aufgabe wird erfindungsgemäß durch eine Zusammensetzung mit den Merkmalen des Anspruchs 1 gelöst.

Vorteilhafte Weiterbildungen sind Gegenstand der Unteransprüche.

Die erfindungsgemäße Zusammensetzung enthält folgende Inhaltsstoffe (INCI): Olea Europaea Fruit Oil, Urea, Silver sowie Octyldodecanol, Echium Plantagineum Seed Oil, Cardiospermum Halicacabum Leaf Extract, Helianthus Annuus Seed Oil Unsaponifiables, Tocopherol. Das Olivenöl pflegt die Haut nachhaltig, so dass sie sich wieder glatt und geschmeidig anfühlt. Urea dient als Feuchtigkeitsspender bzw. als Feuchthaltesubstanz und macht die erfindungsgemäße Zusammensetzung auch für die Verwendung bei Kleinkindern geeignet. Das in der Zusammensetzung enthaltene Silber beseitigt insbesondere bei stark beanspruchter Haut Keime sanft und vermindert weitere Belastungen und Reizungen der Haut. Die Inhaltsstoffe Octyldodecanol, Echium Plantagineum Seed Oil, Cardiospermum Halicacabum Leaf Extract, Helianthus Annuus Seed Oil Unsaponifiables, Tocopherol dienen als Juckreizstiller. Die erfindungsgemäße Zusammensetzung ist ein dermatologisches Produkt zur intensiven Pflege sehr trockener, gereizter und juckender Haut. Es kann auch Therapie begleitend bei Neurodermitis und Psoriasis zur Anwendung kommen. Eine wirkstoffreiche Öl-in-Wasser-Emulsion wurde speziell für die stark beanspruchte Haut entwickelt, um Reizungen zu mindern und die Haut wieder ins Gleichgewicht zu bringen. Sie versorgt sehr trockene, gereizte und schuppige Haut intensiv mit Haut beruhigenden Wirkstoffen und Feuchtigkeit. Insofern kann die erfindungsgemäße Zusammensetzung besonders vorteilhaft als dermatologische Akut-Salbe eingesetzt werden. Sie lindert Juckreiz und ist aufgrund der besonderen, einzigartigen Wirkstoffkombination schnell wirkend. Sie versorgt sehr trockene, gereizte und juckende Haut intensiv mit pflegenden Lipiden und Feuchtigkeit.

Gemäß einer besonders bevorzugten Ausführungsform der Erfindung liegen die Inhaltsstoffe in der Zusammensetzung in folgenden Mengen vor:

| Inhaltsstoff (INCl) | Menge [g/100 g] | bevorzugte Menge [g/100 g] |
|---|---|---|
| Olea Europaea Fruit Oil | 2 - 8 | 5 |
| Urea | 1 - 10 | 2 |
| Silver | 0,1 - 0,3 | 0,1 |
| Octyldodecanol, Echium | 1 - 5 | 4 |
| Plantagineum Seed Oil, | | |
| Cardiospermum Halicacabum | | |
| Leaf Extract, Helianthus Annuus | | |
| Seed Oil Unsaponifiables, | | |
| Tocopherol | | |

Es wurde überraschend gefunden, dass die genannte Wirkstoffkombination mit den Inhaltsstoffen in den vorgenannten Mengen in der Lage ist, besonders schnell Haut pflegend und Reiz mindernd zu wirken.

Gemäß einer anderen Weiterbildung der Erfindung enthält die erfindungsgemäße Zusammensetzung folgende weitere Inhaltsstoffe (INCI): Aqua; Caprylic/Capric Triglyceride; Butyrospermum Parkii Butter; Glycerin; Cetearyl Alcohol; Panthenol; Glyceryl Stearate Citrate; Sodium Lactate (60 Gew.-%), Aqua (40 Gew.-%); Phenoxyethanol, Ethylhexylglycerin; Cera Alba; Lactic Acid; Sucrose Stearate; Olea Europaea Leaf Extract (65 Gew.-%), Aqua (35 Gew.-%); Xanthan Gum; Sodium Hydroxide; Carbomer; Heliotropine; Tocopherol, Hydrogenated Palm Glycerides Citrate, wobei die weiteren Inhaltsstoffe vorzugsweise in den nachfolgend genannten Mengen vorliegen:

| Inhaltsstoff (INCI) | Menge [g/100 g] | bevorzugte Menge [g/100 g] |
|---|---|---|
| Aqua | 50 - 70 | 60,95 |
| Caprylic/Capric Triglyceride | 2 - 8 | 5,00 |
| Butyrospermum Parkii Butter | 2 - 8 | 5,00 |
| Glycerin | 2 - 8 | 4,25 |
| Cetearyl Alcohol | 2 - 5 | 5,00 |
| Panthenol | 0,1 - 5 | 1,50 |
| Glyceryl Stearate Citrate | 1 - 6 | 2,00 |
| Sodium Lactate (60 Gew.-%), Aqua (40 Gew.-%) | 1 - 3 | 1,00 |
| Phenoxyethanol, Ethylhexylglycerin | 0,3 - 1 | 1,00 |
| Cera Alba | 0,2 - 5 | 1,00 |
| Lactic Acid | 0,3 - 1 | 0,40 |
| Sucrose Stearate | 0,3 - 5 | 0,50 |
| Olea Europaea Leaf Extract (65 Gew.-%), Aqua (35 Gew.-%) | 0,1 - 0,5 | 0,50 |
| Xanthan Gum | 0,2 - 1,0 | 0,40 |
| Sodium Hydroxide | 0,1 - 0,3 | 0,15 |
| Carbomer | 0,05 - 1,0 | 0,10 |
| Heliotropine | 0,05 - 0,2 | 0,10 |
| Tocopherol, Hydrogenated Palm Glycerides Citrate | 0,03 - 0,8 | 0,05 |

Aqua dient als Lösungsmittel, Caprylic/Capric Triglyceride als Emollient, d.h. als Mittel, das Körpergewebe weicher und geschmeidiger macht. Butyrospermum Parkii Butter dient als pflegende Butter, Glycerin als Feuchtigkeit spendende Substanz, Cetearyl Alcohol als Emulgator, Panthenol als Haut beruhigender Inhaltsstoff, Glyceryl Stearate Citrate ebenfalls als Emulgator, Sodiumlactat in wässriger Lösung als Neutralisationsmittel, Phenoxyethanol zur Konservierung und Ethylhexylglycerin als Feuchtigkeitsspender, Cera Alba als Konsistenzgeber, Lactic Acid als Neutralisationsmittel, Sucrose Stearate als Emulgator, Olea Europaea Leaf Extract in wässriger Lösung als antioxidativ wirkendes Mittel. Xanthan Gum wirkt als Verdicker, Sodium Hydroxide als Neutralisationsmittel, Carbomer als Gelbildner, Heliotropine als entzündungshemmendes Mittel und Tocopherol sowie Hydrogenated Palm Glycerides Citrate ebenfalls als antioxidativ wirkendes Mittel. Diese Inhaltsstoffe tragen mit zu der einzigartigen Wirkung der erfindungsgemäßen Zusammensetzung als schnell wirkendes, Haut pflegendes und Reiz minderndes Mittel bei.

Vorteilhafterweise ist der Inhaltsstoff (INCI) Silver der Inhaltsstoff Micro Silver vorzugsweise mit einer mittleren Partikelgröße von 1 bis 100 µm. Insofern können die Silberpartikel auf der einen Seite auf der Haut einen Schutzfilm ausbilden und dort die unter Neurodermitis auftretenden Probleme lindern, andererseits sind die Silberpartikel daran gehindert, in tiefe Hautschichten einzudringen und im menschlichen Körper zu sogenannten Silberablagerungen zu führen. Die Silberpartikel haben also eine Größe, die sie daran hindert, über die Blutbahn vom Körper aufgenommen zu werden.

Ausführungsbeispiele der Erfindung werden nachfolgend erläutert, wobei alle beschriebenen Merkmale für sich oder in beliebiger Kombination den Gegenstand der vorliegenden Erfindung unabhängig von ihrer Zusammenfassung in den Ansprüchen oder deren Rückbeziehung bilden.

Die erfindungsgemäße Zusammensetzung liegt insbesondere aber nicht ausschließlich als dermatologische Salbe vor. Sie enthält als wesentliche Inhaltsstoffe gemäß der international üblichen Nomenklatur "International Nomenclature Cosmetic Ingredients (INCI)": Olea Europaea Fruit Oil, Urea, Silver sowie Octyldodecanol, Echium Plantagineum Seed Oil, Cardiospermum Halicacabum Leaf Extract, Helianthus Annuus Seed Oil Unsaponifiables, Tocopherol.

Das genannte Olea Europaea Fruit Oil kann als Olivenöl nativ vorzugsweise mit einem Anteil von 3,00 Gew.-%, als Olivenöl Buti vorzugsweise mit einem Anteil von 1,00 Gew.-% und als Olivenöl Sessa vorzugsweise mit einem Anteil von 1,00 Gew.-% vorliegen, so dass sich insgesamt ein Anteil des Olivenöls in der erfindungsgemäßen Zusammensetzung von vorzugsweise 5,00 Gew.-% ergibt. Das genannte Olivenöl ist vorzugsweise wertvollstes toskanisches Olivenöl. Es kommt in der erfindungsgemäßen Zusammensetzung als pflegendes Öl zum Einsatz.

Urea, Harnstoff, ist ein natürlicher Feuchthaltestoff, welcher demnach in der Lage ist, der Haut Feuchtigkeit zuzuführen und die Feuchtigkeit auch zu speichern.

Silver hat, wie zuvor erwähnt, eine antibakterielle Wirkung und ist insofern in der Lage, auf der Haut befindliche Keime und Bakterien zu beseitigen, so dass deren nachteilige Wirkung auf den menschlichen Körper verringert bzw. ausgeschlossen ist.

Die Inhaltsstoffe Octyldodecanol, Echium Plantagineum Seed Oil, Cardiospermum Halicacabum Leaf Extract, Helianthus Annuus Seed Oil Unsaponifiables, Tocopherol sind im Handel beispielsweise unter der Bezeichnung Defensil^{®} erhältlich, wobei in diesem Produkt Octyldodecanol mit einem Anteil von >50 Gew.-%, Echium Plantagineum Seed Oil mit einem Anteil von etwa 5 Gew.-%, Cardiospermum Halicacabum Leaf Extract mit einem Anteil von etwa 1 Gew.-%, Helianthus Annuus Seed Oil Unsaponifiables mit einem Anteil von etwa 5 Gew.-%, Tocopherol mit einem Anteil von etwa 0,05 Gew.-% vorliegen. Dieses Produkt kann über die Firma Rahn GmbH, Frankfurt am Main, bezogen werden.

Gemäß nachfolgender Tabelle liegen die vorgenannten Inhaltsstoffe in der Zusammensetzung in folgenden Mengen vor:

| Inhaltsstoff (INCI) | Menge [g/100 g] | bevorzugte Menge [g/100 g] |
|---|---|---|
| Olea Europaea Fruit Oil | 2 - 8 | 5 |
| Urea | 1 - 10 | 2 |
| Silver | 0,1 - 0,3 | 0,1 |
| Octyldodecanol, Echium | 1 - 5 | 4 |
| Plantagineum Seed Oil, | | |
| Cardiospermum Halicacabum | | |
| Leaf Extract, Helianthus Annuus | | |
| Seed Oil Unsaponifiables, | | |
| Tocopherol | | |

Damit ist klar, dass die einzelnen Mengen bzw. Anteile der genannten Inhaltsstoffe in der erfindungsgemäßen Zusammensetzung Schwankungen in den genannten Bereichen unterliegen und insofern variieren können.

Das gilt in analoger Weise auch für die folgenden weiteren Inhaltsstoffe (INCI): Aqua; Caprylic/Capric Triglyceride; Butyrospermum Parkii Butter; Glycerin; Cetearyl Alcohol; Panthenol; Glyceryl Stearate Citrate; Sodium Lactate (60 Gew.-%), Aqua (40 Gew.-%); Phenoxyethanol, Ethylhexylglycerin; Cera Alba; Lactic Acid; Sucrose Stearate; Olea Europaea Leaf Extract (65 Gew.-%), Aqua (35 Gew.-%); Xanthan Gum; Sodium Hydroxide; Carbomer; Heliotropine; Tocopherol, Hydrogenated Palm Glycerides Citrate.

Die Anteile bzw. Mengen der vorgenannten weiteren Inhaltsstoffe können in der Zusammensetzung innerhalb weiter Bereiche schwanken. Diese sind ebenso wie die bevorzugten Mengen in Bezug auf die weiteren Inhaltsstoffe in der nachfolgenden Tabelle angegeben.

| Inhaltsstoff (INCI) | Menge [g/100 g] | bevorzugte Menge [g/100 g] |
|---|---|---|
| Aqua | 50 - 70 | 60,95 |
| Caprylic/Capric Triglyceride | 2 - 8 | 5,00 |
| Butyrospermum Parkii Butter | 2 - 8 | 5,00 |
| Glycerin | 2 - 8 | 4,25 |
| Cetearyl Alcohol | 2 - 5 | 5,00 |
| Panthenol | 0,1 - 5 | 1,50 |
| Glyceryl Stearate Citrate | 1 - 6 | 2,00 |
| Sodium Lactate (60 Gew.-%), Aqua (40 Gew.-%) | 1 - 3 | 1,00 |
| Phenoxyethanol, Ethylhexylglycerin | 0,3 - 1 | 1,00 |
| Cera Alba | 0,2 - 5 | 1,00 |
| Lactic Acid | 0,3 - 1 | 0,40 |
| Sucrose Stearate | 0,3 - 5 | 0,50 |
| Olea Europaea Leaf Extract (65 Gew.-%), Aqua (35 Gew.-%) | 0,1 - 0,5 | 0,50 |
| Xanthan Gum | 0,2 - 1,0 | 0,40 |
| Sodium Hydroxide | 0,1 - 0,3 | 0,15 |
| Carbomer | 0,05 - 1,0 | 0,10 |
| Heliotropine | 0,05 - 0,2 | 0,10 |
| Tocopherol, Hydrogenated Palm Glycerides Citrate | 0,03 - 0,8 | 0,05 |

Die erfindungsgemäße Zusammensetzung dient zur intensiven Pflege sehr trockener, gereizter, schuppiger und juckender Haut. Sie ist ohne Parfum, ohne Farbstoffe, ohne Silicon- und Paraffinöl hergestellt und kann als Lotion, Creme, Emulsion, Salbe, Gel oder Suspension, vorzugsweise als Körpercreme und -balsam, formuliert werden.

Wie erwähnt, ist der Inhaltsstoff (INCI) Silver vorzugsweise Micro Silver mit einer mittleren Partikelgröße von 1 bis 100 µm.

Eine besonders bevorzugte Ausführungsform der erfindungsgemäßen Zusammensetzung ist in der nachfolgenden Tabelle angegeben, in der der Vollständigkeit halber neben den einzelnen INCI-Bezeichnungen auch Handelsnamen der entsprechenden Stoffe angegeben sind. Es wird ausdrücklich darauf hingewiesen, dass einige der Handelsnamen als Marke registriert sein können.

| Lfd. Nr. | Handelsname | INCI - Bezeichnung | Menge [g/100 g] |
|---|---|---|---|
| 1 | Wasser | Aqua | 60,95 |
| 2 | Tegosoft CT | Caprylic/Capric Triglyceride | 5,00 |
| 3 | Lipex Shea, Sheabutter | Butyrospermum Parkii Butter | 5,00 |
| 4 | Glycerin 86% | Glycerin | 4,25 |
| 5 | Tego Alkanol 6855 | Cetearyl Alcohol | 5,00 |
| 6 | Defensil | Octyldodecanol (>50%), Echium Plantagineum Seed Oil (5%), Cardiospermum Halicacabum Leaf Extract (1%), Helianthus Annuus Seed Oil Un-saponifiables (5%), Tocopherol (0,05%) | 4,00 |
| 7 | Olivenöl native | Olea Europaea Fruit Oil | 3,00 |
| 8 | Harnstoff | Urea | 2,00 |
| 9 | D-Panthenol 75L | Panthenol | 1,50 |
| 10 | Imwitor 372 P | Glyceryl Stearate Citrate | 2,00 |
| 11 | Natriumlactatlösung 60% | Sodium Lactate (60%), Aqua (40%) | 1,00 |
| 12 | Olivenöl Buti | Olea Europaea Fruit Oil | 1,00 |
| 13 | Euxyl PE 9010 | Phenoxyethanol, Ethylhexylglycerin | 1,00 |
| 14 | Bienenwachs, gebl. | Cera Alba | 1,00 |
| 15 | Olivenöl Sessa | Olea Europaea Fruit Oil | 1,00 |
| 16 | Milchsäure | Lactic Acid | 0,40 |
| 17 | Sisterna SP 70C | Sucrose Stearate | 0,50 |
| 18 | Eurol BT, Olivenblatt-extrakt | Olea Europaea Leaf Extract (65%), Aqua (35%) | 0,50 |
| 19 | Keltrol CG SFT | Xanthan Gum | 0,40 |
| 20 | Natriumhydroxid, Natronlauge | Sodium Hydroxide | 0,15 |
| 21 | Carbopol Ultrez 10 | Carbomer | 0,10 |
| 22 | Micro Silver BG (IMPAG) | Silver | 0,10 |
| 23 | Heliotropin | Heliotropine | 0,10 |
| 24 | Controx KS | Tocopherol, Hydrogenated Palm Glycerides Citrate | 0,05 |

Damit ist eine schnell wirksame, Haut pflegende und Reiz mindernde Zusammensetzung geschaffen, welche auch für die tägliche Körperpflege eingesetzt werden kann.

## Patentansprüche

1. Zusammensetzung, insbesondere dermatologische Salbe, enthaltend folgende Inhaltsstoffe (INCl):
Olea Europaea Fruit Oil,
Urea,
Silver sowie
Octyldodecanol, Echium Plantagineum Seed Oil, Cardiospermum Halicacabum Leaf Extract, Helianthus Annuus Seed Oil Unsaponifiables, Tocopherol.

2. Zusammensetzung nach Anspruch 1, **dadurch gekennzeichnet, dass** die Inhaltsstoffe in der Zusammensetzung in folgenden Mengen vorliegen:
| Inhaltsstoff (INCI) | Menge [g/100 g] | bevorzugte Menge [g/100 g] |
|---|---|---|
| Olea Europaea Fruit Oil | 2 - 8 | 5 |
| Urea | 1 - 10 | 2 |
| Silver | 0,1 - 0,3 | 0,1 |
| Octyldodecanol, Echium | 1 - 5 | 4 |
| Plantagineum Seed Oil, | | |
| Cardiospermum Halicacabum | | |
| Leaf Extract, Helianthus Annuus | | |
| Seed Oil Unsaponifiables, | | |
| Tocopherol | | |

3. Zusammensetzung nach Anspruch 1 oder 2, **dadurch gekennzeichnet, dass** folgende weitere Inhaltsstoffe (INCI): Aqua; Caprylic/Capric Triglyceride; Butyrospermum Parkii Butter; Glycerin; Cetearyl Alcohol; Panthenol; Glyceryl Stearate Citrate; Sodium Lactate (60 Gew.-%), Aqua (40 Gew.-%); Phenoxyethanol, Ethylhexylglycerin; Cera Alba; Lactic Acid; Sucrose Stearate; Olea Europaea Leaf Extract (65 Gew.-%), Aqua (35 Gew.-%); Xanthan Gum; Sodium Hydroxide; Carbomer; Heliotropine; Tocopherol, Hydrogenated Palm Glycerides Citrate.

4. Zusammensetzung nach Anspruch 3, **dadurch gekennzeichnet, dass** die weiteren Inhaltsstoffe in der Zusammensetzung in folgenden Mengen vorliegen:
| Inhaltsstoff (INCI) | Menge [g/100 g] | bevorzugte Menge [g/100 g] |
|---|---|---|
| Aqua | 50 - 70 | 60,95 |
| Caprylic/Capric Triglyceride | 2 - 8 | 5,00 |
| Butyrospermum Parkii Butter | 2 - 8 | 5,00 |
| Glycerin | 2 - 8 | 4,25 |
| Cetearyl Alcohol | 2 - 5 | 5,00 |
| Panthenol | 0,1 - 5 | 1,50 |
| Glyceryl Stearate Citrate | 1 - 6 | 2,00 |
| Sodium Lactate (60 Gew.-%), Aqua (40 Gew.-%) | 1 - 3 | 1,00 |
| Phenoxyethanol, Ethylhexylglycerin | 0,3 - 1 | 1,00 |
| Cera Alba | 0,2 - 5 | 1,00 |
| Lactic Acid | 0,3 - 1 | 0,40 |
| Sucrose Stearate | 0,3 - 5 | 0,50 |
| Olea Europaea Leaf Extract (65 Gew.-%), Aqua (35 Gew.-%) | 0,1 - 0,5 | 0,50 |
| Xanthan Gum | 0,2 - 1,0 | 0,40 |
| Sodium Hydroxide | 0,1 - 0,3 | 0,15 |
| Carbomer | 0,05 - 1,0 | 0,10 |
| Heliotropine | 0,05 - 0,2 | 0,10 |
| Tocopherol, Hydrogenated Palm Glycerides Citrate | 0,03 - 0,8 | 0,05 |

5. Zusammensetzung nach einem der Ansprüche 1 bis 4, **dadurch gekennzeichnet, dass** der Inhaltsstoff (INCI) Silver der Inhaltsstoff Micro Silver vorzugsweise mit einer mittleren Partikelgröße von 1 bis 100 µm ist.
